Europäisches Patentamt

European Patent Office

Office européen des brevets

(19)

(11) Publication number: **0 637 745 A1**

(12) **EUROPEAN PATENT APPLICATION**
published in accordance with Art. 158(3) EPC

(21) Application number: 94907691.3

(22) Date of filing: **25.02.94**

(51) Int. Cl.⁶: **G01N 21/89**

(86) International application number:
**PCT/JP94/00309**

(87) International publication number:
**WO 94/19680 (01.09.94 94/20)**

(30) Priority: 25.02.93 JP 37118/93
25.02.93 JP 37119/93
25.02.93 JP 37120/93
18.10.93 PCT/JP93/01491
18.02.94 JP 21279/94
18.02.94 JP 21280/94

(43) Date of publication of application:
**08.02.95 Bulletin 95/06**

(84) Designated Contracting States:
**BE DE FR GB IT NL PT**

(71) Applicant: **NIKKISO CO., LTD.**
**No. 43-2, Ebisu 3-chome**
**Shibuya-ku**
**Tokyo 150 (JP)**
Applicant: **SHIN-ETSU CHEMICAL CO., LTD.**
**Asahitokai Bldg.**
**6-1**
**Otemachi 2-chome**
**Chiyoda-ku**
**Tokyo 100 (JP)**

(72) Inventor: **KAMINAGA, Kenzo,**
**Higashimurayama Plant of**
**Nikkiso Co., Ltd.,**
**16-2, Noguchi-cho 2-chome**
**Higashimurayama-shi,**
**Tokyo 189 (JP)**
Inventor: **SUZUKI, Masaaki Higashimurayama**
**Plant of**
**Nikkiso Co., Ltd,**
**16-2, Noguchi-cho 2-chome**
**Higashimurayama-shi,**
**Tokyo 189 (JP)**
Inventor: **KIMURA, Takashi Higashimurayama**
**Plant of**

Nikkiso Co., Ltd,
16-2, Noguchi-cho 2-chome
Higashimurayama-shi,
Tokyo 189 (JP)
Inventor: **KATO, Keisuke Higashimurayama**
**Plant of**
Nikkiso Co., Ltd,
16-2, Noguchi-cho 2-chome
Higashimurayama-shi,
Tokyo 189 (JP)
Inventor: **NOGAMI, Yuji**
Nikkiso Co., Ltd,
43-2, Ebisu 3-chome
Shibuya-ku,
Tokyo 150 (JP)
Inventor: **HIRAKURI, Yoshihisa**
**Higashimurayama Plant of**
Nikkiso Co., Ltd,
16-2, Noguchi-cho 2-chome
Higashimurayama-shi,
Tokyo 189 (JP)
Inventor: **CHINO, Takashi**
Nikkiso Co., Ltd,
43-2, Ebisu 3-chome
Shibuya-ku,
Tokyo 150 (JP)
Inventor: **KAWAMURA, Takao**
Nikkiso Co., Ltd,
43-2, Ebisu 3-chome
Shibuya-ku,
Tokyo 150 (JP)
Inventor: **TSUTSUMI, Atsushi**
**Higashimurayama Plant of**
Nikkiso Co., Ltd,
16-2, Noguchi-cho 2-chome
Higashimurayama-shi,
Tokyo 189 (JP)

EP 0 637 745 A1

Rank Xerox (UK) Business Services
(3.10/3.09/3.3.4)

Inventor: **TAKEUCHI, Masaru**
**6-25, Doainishi 4-chome**
**Hasaki-machi**
**Kashima-gun**
**Ibaraki 314-03 (JP)**
Inventor: **YOSHIKOSHI, Hideo**
**7-16, Doainishi 2-chome**
**Hasaki-machi**
**Kashima-gun**

**Ibaraki 314-03 (JP)**

(74) Representative: **Smulders, Theodorus A.H.J., Ir.**
**Vereenigde Octrooibureaux**
**Nieuwe Parklaan 97**
**NL-2587 BN 's-Gravenhage (NL)**

(54) **AUTOMATIC INSPECTION APPARATUS FOR FISH EYE IN RESIN, AND APPARATUS AND METHOD FOR PRODUCING RESIN SHEET FOR INSPECTION.**

(57) A shaft support member of at least one (12) of a pair of kneading rolls (11, 12) opposing each other with a very small gap between them and forming a valley portion, in which a resin charged from above stays, is connected to a driving source which changes a shaft gap, and a pair of weir plates (15b) having a shape substantially in conformity with the shape of the valley portion and adjacent to the kneading rolls (11, 12) without coming into contact with them are connected to a moving driving source in the axial direction of the kneading rolls (11, 12) while being spaced apart by a substantial effective width of the kneading rolls (11, 12). A sheet frame supply source supplies sheet frames (40) one by one toward the valley portion. Conveyor means (57, 58) for an inspection sheet is disposed at a discharge position of the inspection sheet, which is formed by bonding a resin sheet (37) kneaded and formed by a pair of kneading rolls (11, 12) and bonded to the sheet frame (40), and a table (59) for the inspection sheet and an imaging apparatus including a light source (64) and a video camera (65) are successively disposed downstream of the conveyor means (57, 58).

## FIG.2

Technical Field

The present invention relates to an automatic inspection apparatus for fisheyes in such thermoplastic resins as a vinyl chloride resin to an apparatus which automatically manufactures the resin sheets to be specimens for such inspection and to the process for automatic manufacture of the resin sheets to be specimens for the same inspection.

Background Art

Ideally any synthetic resins should allow no mingling of any foreign matters nor any such heterogenous mixtures as non-gelated particles. However fabrication of any synthetic resins perfectly homogeneous is a task of extreme difficulty. In most cases therefore trying in vain to manufacture such perfect products would be to seek after over-quality, which will lead normally to higher cost. In this line of thinking most of synthetic resin manufacturers are inspecting quantitatively foreign matters and non-gelated particles in the resins to furnish the secondary transformation with the synthetic resins whose quality could be improved therethrough into that corresponding to the quality of the final products required.

The control of non-gelated particles and other foreign matters in synthetic resins, called "fisheye" inspection, has been achieved visually and optically. More in concrete, the synthetic resins in powdered form are spread homogeneously and observed visually (by naked eyes) to detect any heterogeneous or non-gelated particles or else photographed and measured by means of a video camera. Sometimes the synthetic resins are formed into sheets to measure any foreign matters and fisheyes. Depending upon more or less fisheyes the synthetic resins corresponding to the production lot are provided to some appropriate uses.

Conventionally the resins sheets applied to the foregoing examination have been manufactured by roll mill, a kneading machine. Provided in the roll mill is a pair of heating type kneading rolls the distance between which can be adjusted. The two kneading rolls are heated beforehand to a prescribed temperature in terms of the melting point of the resin to be processed, and the gap therebetween is regulated in match with the thickness of the resin sheet to be formed. One of the kneading rolls will be rotated at a high speed, while the other, at a low speed. When the compound for synthetic resin is put into the valley-formed portion between the two rolls, it melts down by the heat radiated from the respective rolls and goes to be kneaded by being crashed or sheared therebetween. The synthetic resin will then be wound up in sheet form on the roll turning under a high speed. When the sheet of synthetic resin will become homogeneous or nearly so, a part of the sheet will be cut off the roll to be measured in thickness by means of a dial gage. If the sheet presents desired thickness, then it will be cut out along the longitudinal direction of the roll and pinched out.

Thus the fisheyes of the sheet prepared in the kneading machine is measured by an inspection instrument provided with a video camera.

In the traditional fisheye inspection process thus far described, all the operations have been manually exercised; operations such as reapplication of the synthetic resin compound which falls down from under the fine gap between the kneading rolls, kneading of the sheet wrapped up around the rolls, measurement of the sheet thickness, cutting of the sheet wound upon the kneading roll, spreading and setting of the sheet upon the sampling table, and finally the mounting of the sheet for inspection on this same table. The respective operations require skilled workers. Because certain differences in resin sheets for inspection derive from these workers who are subtly different from each other though they may be duly qualified in skill at a standard level, even the synthetic resins in a same production lot often give rise to some, though little, deviation in resin sheets for inspection thereby causing different inspection results. Another demerit of the conventional operations is that the workers have been exposed to the risk of injury or burn at their hands or fingers due to the fact that the kneading rolls are turning at substantially high temperatures.

The present invention, designed to resolve these problems, provides an automatic fisheye inspection apparatus excellent in safety, intended to perform efficiently and automatically the inspection of the fisheyes in synthetic resins. Any operators can therefore complete the inspection with good reproducibility not manually but automatically without resorting to any skill nor to any substantial manpower. Further this invention can provide both resins sheets manufacturing apparatus and process, superb in safety, to manufacture automatically and under excellent efficiency quality resins sheets for inspection.

Disclosure of the Invention

The automatic inspection apparatus for fisheyes in resins according to this invention is characterized in that the axial support member of at least one roll of a pair of kneading rolls, opposed to each other with a fine gap therebetween, which delimit a valley-formed portion where the resin supplied from above the rolls stay, is coupled with a driving source which displaces the axial gap, which a pair of end plates under a form almost going along with said valley, nearing to, but not in contact with the kneading rolls, are coupled with a moving driving source in the same direction with the axial one of said kneading rolls apart from a substantially effective length of these rolls, that the apparatus has a sheet frame supplying source which sends the sheet frames one by one toward said valley-formed portion, and that at a position where the sheets for inspection that are formed by the resin sheet formed by kneading operation of said pair of kneading rolls and stuck onto the sheet frame, there are provided a means to carry said sheets for inspection and then a photographing device including a set-up table for said sheets for inspection, a light source and a video camera.

The sheet manufacturing apparatus for inspection of resins according to this invention is characterized in that the axial support member of at least one roll of a pair of those kneading rolls, opposed to each other with a fine gap therebetween, which delimit the valley-formed portion where the resin introduced from above said rolls will stay, is coupled to a driving source which displaces the axial gap, that a pair of end plates under such geometrical form as almost along the valley portion, nearing to but not in contact with the kneading rolls, are coupled with a moving drive source in the same direction as the axial direction of said kneading rolls apart from the substantially effective length of said rolls and that there is provided a sheet frame supplying source which sends the frames one by one toward the aforesaid valley portion.

The manufacturing process of the resin sheets for inspection by this invention is characterized in that a pair of heated kneading rolls are opposed to each other with a fine gap therebetween, one of the rolls being rotated at a high speed and the other at a low one both in the direction to draw the sheets from above into between these rolls in such a way that the resin as introduced from above the rolls into the valley portion therebetween can be molten down and made to adhere onto the roll rotating at a high speed, the resin drooping from between the fine gap being received on a belt, transferred and made to be in contact with the highly rotating roll to be molten down and adhered thereto, that after kneading the resin which was molten down and adhered by varying the fine gap between the pair of kneading rolls and by changing the substantially effective length of the kneading rolls in their axial direction, the fine gap between the pair of rolls is adjusted into prescribed distance, and that the sheet-formed resin on the highly rotating roll, made into specified thickness and width by regulating the substantial effective width of the kneading rolls in their axial direction, is displaced by its contact with the sheet frame.

Brief Description of the Drawings

Figure 1 is a perspective illustration of an embodiment as a whole of the automatic inspection apparatus of fisheyes in resin according to the present invention.

Figure 2 is a side view of the lower portion of the same apparatus which shows the outline of said embodiment.

Figure 3 is a perspective view of the rear lower portion of the apparatus embodied as above.

Figure 4 is a sectional view of the upper portion of said embodiment of the invention.

Figures 5A, 5B, 5C and 5D are the outline drawings which show up the operational processes of the foregoing embodiment of this invention.

Figure 6 is a block diagram of the control circuit of the automatic fisheye inspection apparatus.

Figures 7A, 7B, 7C and 7D are the flow charts which depict the operational processes of the automatic fisheye inspection apparatus.

Figure 8 illustrates a modified embodiment of the configuration for pushing the sheet frame against the resin sheet.

Figure 9 illustrates a modified embodiment of the sheet frame.

Figures 10A and 10B show another modified embodiment of the configuration for pushing the sheet frame against the resin sheet.

Figure 11 represents a modified embodiment of the configuration of the end plate.

Figure 12 depicts a modified embodiment of the configuration in which the resin compound drooping from under the fine gap between the kneading rolls is returned to the upper portion.

Figure 13 represents a modified embodiment of the kneading rolls.

Best Mode of Carrying out the Invention

Now referring to the attached drawings detailed explanation will be given of the embodiments of this invention.

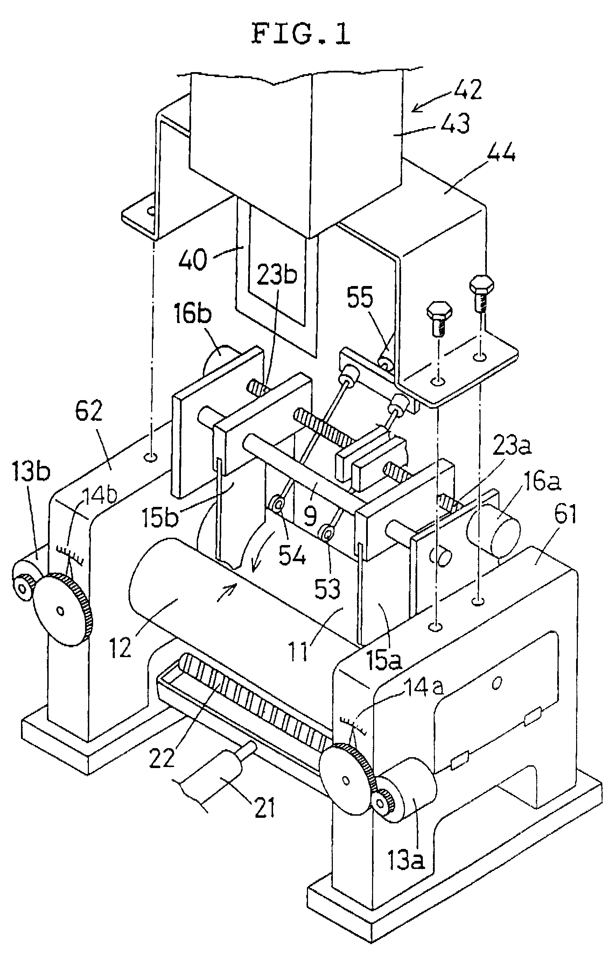

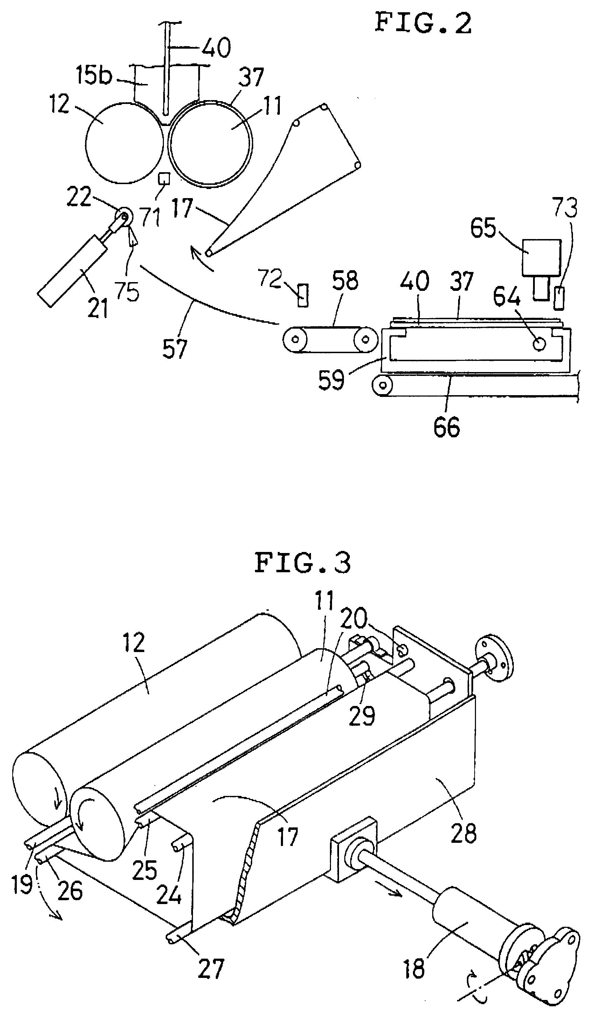

Figure 1 is a perspective view showing the one embodiment as a whole of the sheet manufacturing apparatus for resin inspection to which the present invention is applied. This sheet manufacturing apparatus for resin inspection, together with the inspection portion as shown in Fig. 2, the cross sectional outline drawing of the essential part, constitutes the automatic fisheye inspection apparatus.

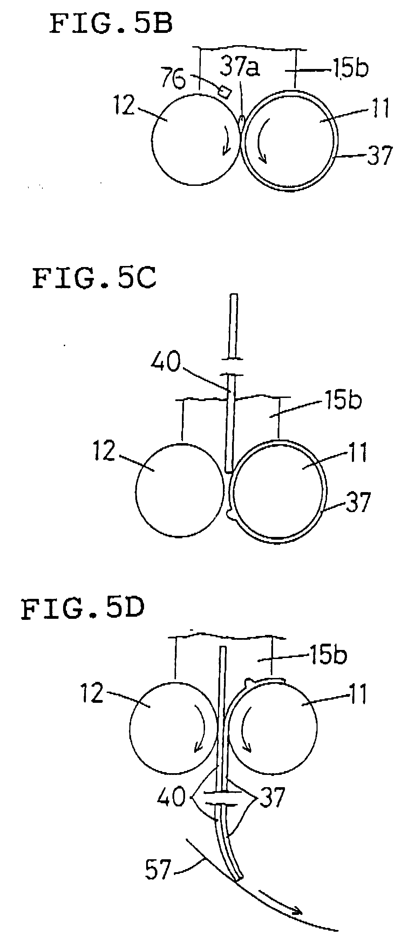

Now an attempt is made to describe the sheet manufacturing apparatus for resin inspection referring to Fig. 1. As has been shown the apparatus is assembled on frames 61 and 62 which are symmetric as a whole. Between the frames 61 and 62, a pair of kneading rolls 11 and 12 are axially supported. These kneading rolls, arranged in parallel, come near and opposed to each other forming a valley-like portion therebetween. Provided between these rolls is a photoelectric sensor 76 (refer to Fig. 5B) for resin bank whose detection range is said valley portion. The respective rotating shafts of the kneading rolls 11 and 12 are coupled with distinct drive motors and the axial support member of the roll 11 is fixed with the frames 61 and 62, while the both sides of the axial support member for the roll 12 are so screwed into the screw rod connected with motors 13a and 13b that the kneading roll 12 displaces by parallel translation as the motors 13a and 13b rotate to come nearer to or go away from the kneading roll 11. The screw rod is provided with indicating pointers 14a and 14b which allow to read out the gap between the rolls 11 and 12 which incorporate high frequency induction coils and are connected with power supply thus enabling to be heated.

A pair of end plates 15a and 15b regulate the extension of the resin as kneaded by the kneading rolls 11 and 12, and the distance between these plates determines the substantial effective width of the rolls 11 and 12. Both end plates 15a and 15b are engaged into a guide bar 9, in a way capable of sliding, and screwed respectively into the screw rods 23a and 23b. The screw rod 23a is coupled with a pulse motor 16a while the screw rod 23b with another pulse motor 16b. It is so designed that when the pulse motors 16a and 16b rotate, the end plates 15a and 15b come nearer to, or go away from each other in such a fashion that the substantially effective width of the kneading rolls 11 and 12 should change accordingly.

In the apparatus shown in Figure 1, an endless belt 17 shown in Fig. 3 is provided at a position not shown here (the other side of Fig. 1). The endless belt 17 has a width almost same with the substantial effective width of the kneading rolls 11 and 12, and suspendedly rotated by the shafts 24, 25, 26 and 27 which are rotatably supported by the belt frame 28. The shift 24 is rotatably supported by the frames 61 and 62, while the shaft 26 is engaged into the circular arc guide groove 29 on the sides of the frames 61 and 62 whose center is the shaft 24. An air cylinder 18 is pivotally mounted on the chassis plate across the frames 61 and 62 so that the cylinder 18 can rotate upward and downward, and the plunger of the cylinder 18 is coupled with the belt frame 28. This mechanism makes it possible that by the action of the air cylinder 18, the endless belt 17 advances and retreats together with the belt frame 28 so that the belt can come in contact with or go away from the roll 11. Further mounted on the belt frame 28 and in contact with the surface of the endless belt 17 is a scraper 20 for the belt, and another scraper 19 for roll 12 is mounted on the surface of this roll. Furthermore in the lower portion of the kneading rolls 11 and 12, a cleaning roll 22 coupled with the air cylinder 21 is telescopically mounted so that the roll 22 can advance to and retire from the position in contact with the rolls 11 and 12. A scraper 75 for cleaning roll 22 is provided on this roll.

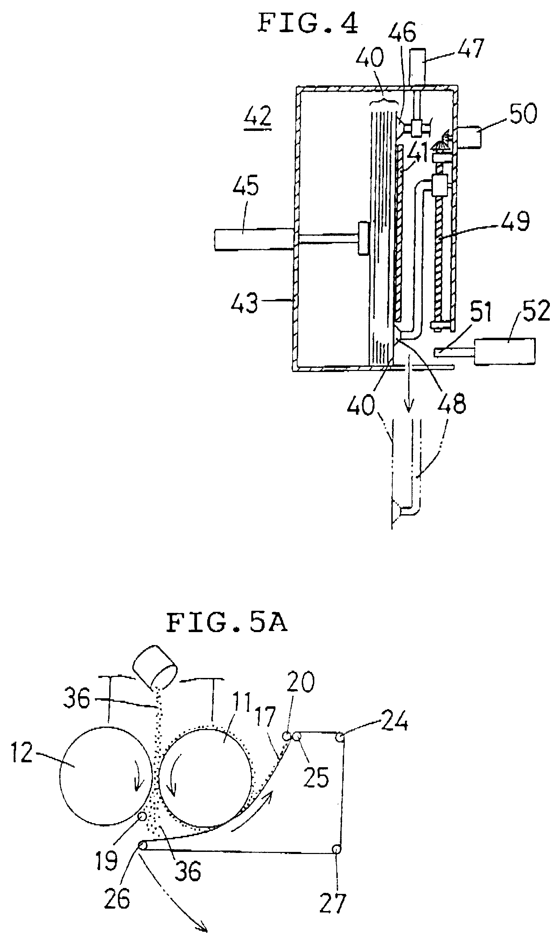

A sheet frame supply source 42 is fixed on the frames 61 and 62 through intermediary of a mount 44. This supply source 42, whose detailed sectional view is given in Figure 4, lets the sheet frames 40 fall one by one toward the valley-shaped portion delimited by the kneading rolls 11 and 12. The major components of the sheet frame supply source 42 are arranged inside the casing 43. Provided inside the casing 43 is a partition plate 41 the lower portion of which is kept open to let the sheets frames 40 fall freely. A great number of cardboard sheet frames 40 are vertically housed against the partition plate 41 where an air cylinder 45 is furnished to hold the sheet frames 40 and the partition plate 41. Above the partition plate 41, an upper suction cap 46 is instilled so that the cap can move up and down in connection with an air cylinder 47. Below the partition plate 41 a lower suction cap 48 is arranged which is screwed into the screw rod 49 and engaged with the guide bar. Because the screw rod 49 is coupled with a motor 50, the lower suction cap can move up and down as the motor 50 rotates. Further below the partition plate 41 a stopper 51 is telescopically provided in connection with the air cylinder 52 in such a way that it can advance and retreat with this cylinder.

Also in the apparatus shown in Figure 1, telescopically provided are a surface displacement meter 53 which comes in contact with the surface of the roll 11 between the end plates 15a and 15b (that is, within the substantial effective width of the roll 11) on the one hand, and another surface displacement meter 54 which comes in contact with the surface of the roll 11 outside the end plates 15a and 15b (that is, outside the substantial effective width of the roll 11), on the other, both in connection with the air cylinder 55. The surface displacement meters 53 and 54 having, as measurers, rollers at their tops, can expand and contract pursuant to fine displacement to output electrically the amount of expansion and contraction. One can know the thickness of the sheet 37 stuck to the surface of the roll 11 from the difference in output values of the expansion and contraction amount.

Figure 2 depicts the inspection portion showing the periphery of the pair of kneading rolls 11 and 12. The sheet manufacturing apparatus for resin inspection as shown in fig. 1, together with the inspection portion as shown in fig. 2, constitutes the automatic fisheye inspection apparatus. A guide plate 57 is installed below the kneading rolls 11 and 12 of the sheet manufacturing apparatus. At a position deviated from just under the rolls 11 and 12 (deep on the other side of the paper), a photoelectric sensor 71 is provided which detects falling resin compound. The guide plate 57 is followed by a belt conveyor 58 in the direct neighborhood of which is installed a photographing device. In this photographing device a light source 64 and a video camera 65 are provided respectively in the lower and upper portions of the sheets for inspection 37 and 40 on the set-up table 59 of the sheets for inspection in such a way that the light passing through the sheet 37 can be photographed. The set-up table 59 of the sheets for inspection, set on the belt conveyor 66 can travel with the sheets for inspection 37 and 40 as transferred from the belt conveyor 58 within the photographing field of view of the video camera 65 which can accordingly photograph in continuous fashion all the sheets 37 running within the field of view one after another. Respectively provided above the belt conveyor 58 and the belt conveyor 66 are sheet No. 1 sensor 72 and sheet No. 2 sensor 73 which detect the sheets for inspection.

The overall face of the sheet 37 can be photographed also when the set-up table 59 of the sheets for inspection is fixed while the light source 64 and the video camera 65 are made to be movable. The same photographing is possible without this movable mechanism if the video camera 65 is of wide angle field of view with higher resolution.

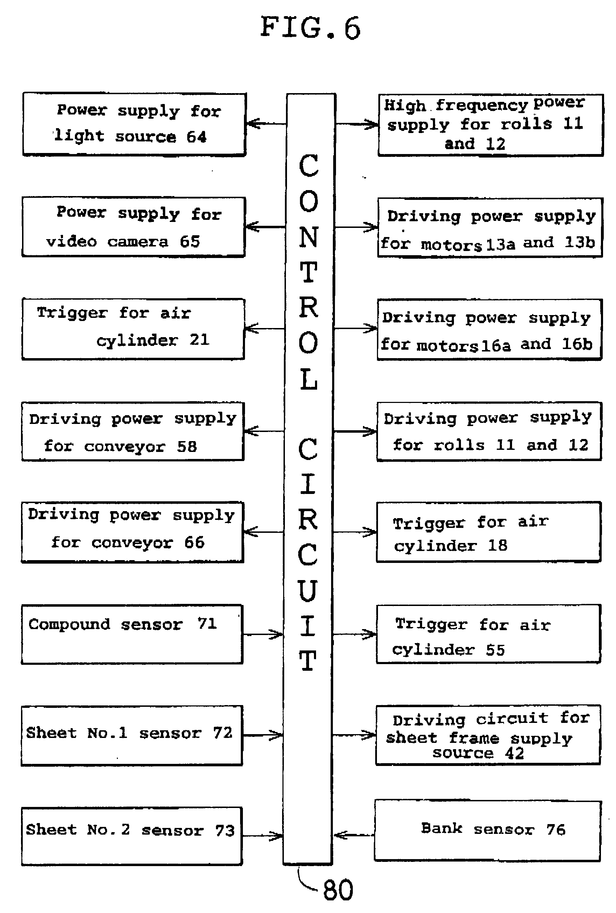

The respective drives of the automatic fisheye inspection apparatus as shown in Figures 1 and 2 are so designed as to be controlled by the control circuit 80 shown in fig. 6. Linked with this control circuit 80 are: a high frequency power supply for rolls 11 and 12, a driving power supply for motors 13a and 13b, another driving power supply for motors 16a and 16b, a motor drive power supply for rotation of the rolls 11 and 12, the trigger of air cylinder 18, the trigger of air cylinder 55, a driving circuit for sheet frame supply source 42, a power supply for the light source 64, another power supply for the video camera 65, the trigger of air cylinder 21, a driving power supply for conveyor 58, and a driving power supply for conveyor 66. Furthermore connected to this control circuit are a sensor 71 for compound, a photoelectric sensor 76 for resin bank, sheet No. 1 sensor 72 and sheet No. 2 sensor 73.

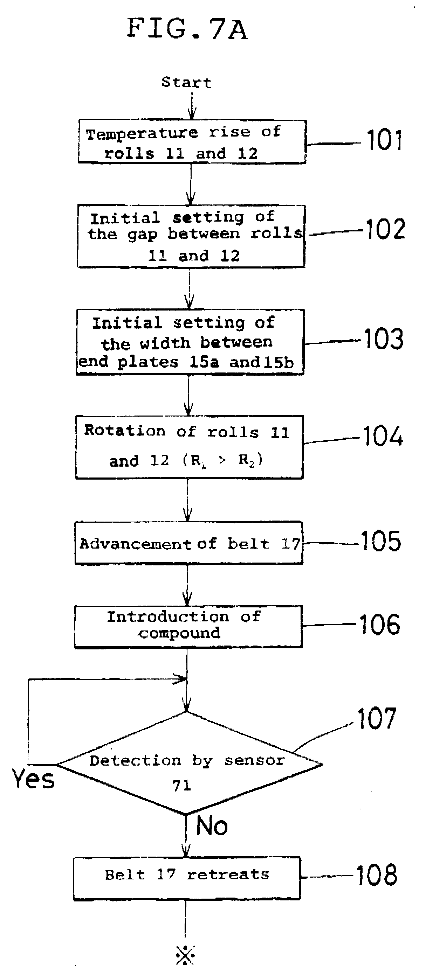

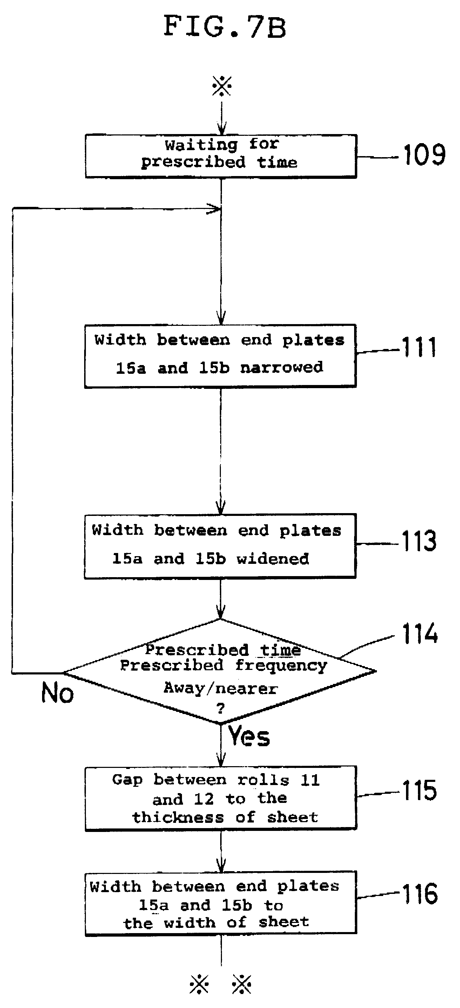

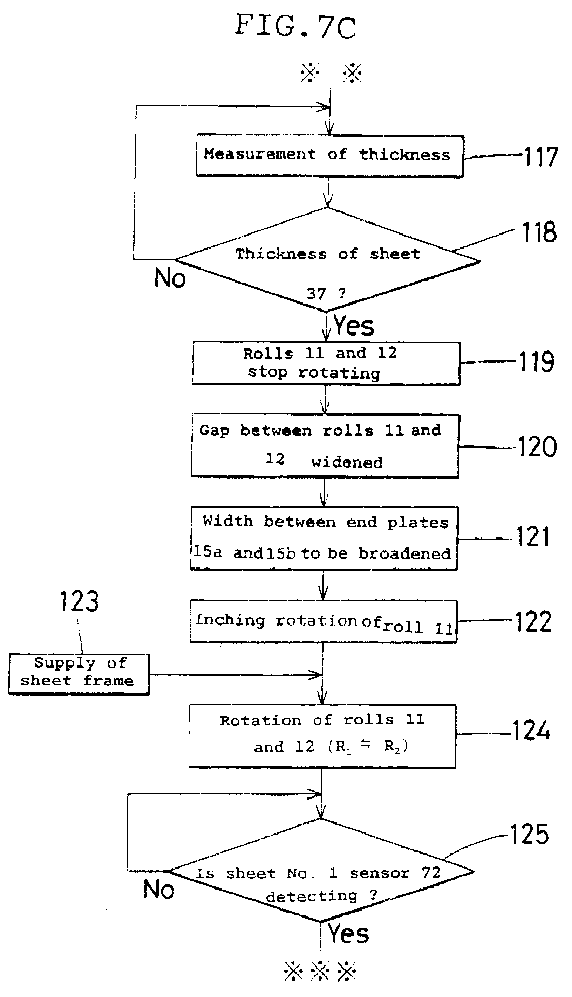

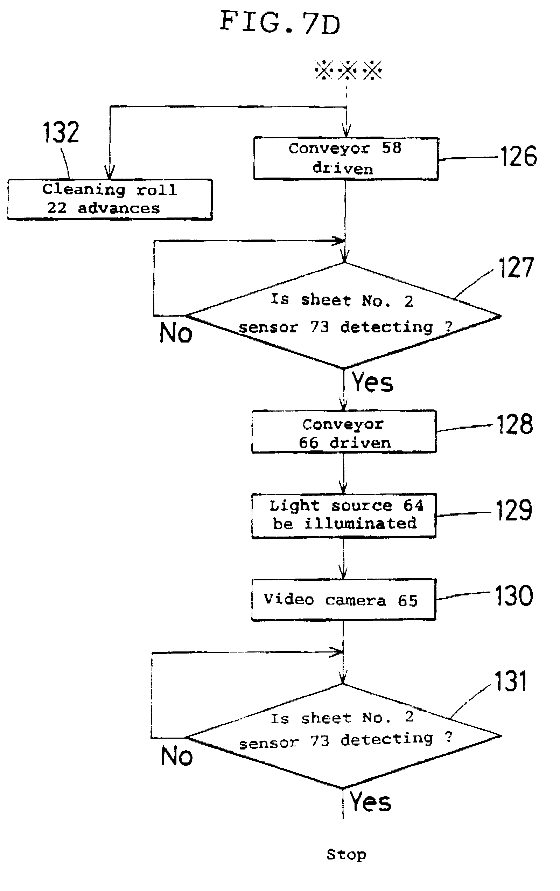

Now an embodiment of the sheet manufacturing apparatus and manufacturing process of the sheets for inspection to which this invention is applied will be discussed hereunder explaining the flow charts, Figures 7A to 7D, referring to the operational processes Figures 5A to 5D of the automatic inspection apparatus of fisheyes in resin according to this invention whose embodiment has been shown in Figures 1 and 2.

At Step 101 the kneading rolls 11 and 12 are raised to a prescribed temperature by electrifying the high frequency induction coils. At Step 102 the motors 13a and 13b shall be started, the values indicated by the pointers 14a and 14b shall be read out to match the gap between the kneading rolls 11 and 12 to the initial setting. At Step 103 the motors 16a and 16b shall be started, and the gap between the end plates 15a and 15b shall be matched with the initial setting. At Step 104 the drive motors for the rolls 11 and 12 shall be started with the rotational speed R1 of the roll 11 being a little higher than that of the other roll 12, R2, when the air cylinder 55 is made to retreat and the surface displacement meters 53 and 54 are made to be both away from the kneading roll 11. At Step 105 the cylinder 18 shall act, and the endless belt 17 shall advance to be in contact with the roll 11, when the endless belt 17 will make its rounds by the frictional drive of the roll 11 which will be turning. On the other hand it is to be checked that the sheet frame 40 has been housed in the casing 43 of the sheet frame supply source 42.

The compound (for example, powdered vinyl chloride resin mixed with a stabilizer and a plasticiser, and if desired, with a coloring agent) for the sample resin to be inspected shall be prepared beforehand. Compound 36 as feedstock is introduced from a beaker into the valley-like portion between the kneading rolls 11 and 12 as shown in Figure 5A under Step 106. Most of the compound 36 is molten down and adheres to the roll 11 which is turning at high speed, but a part of the compound, though very little, sticks to the low speed rotating roll 12 and will be scraped off by the scraper 19 for the roll 12 in contact with the roll surface to fall on the endless belt 17. A part of the compound 36 introduced will pass through the fine gap to fall directly on the endless belt 17. These compounds 36 fallen on the endless belt 17 are conveyed upward by the endless belt going its rounds, pushed against the high speed rotating roll 11 in contact with the belt 17 and then most compounds adhere to this roll 11. The compounds 36 which have not adhered will be conveyed further upward, but will then be scraped away by the scraper 20 for belt, fall into the gap in contact with the endless belt 17, where they will stick to the high speed rotating roll 11. Thus all the compounds 36 introduced will adhere completely to the high speed rotating roll 11. When the compounds 36 cease to fall and that the sensor 71 stops detecting the compounds 36 at step 107, then the air cylinder 18 is made to aspirate at Step 108, when the endless belt 17 centered on the shaft 24, together with the scraper 19 for roll and scraper 20 for belt will reciprocally rotate and retire into the direction of chained arrow away from the kneading roll 11 to open the lower portions of the kneading rolls 11 and 12.

If at Step 109 the high speed roll 11 and low speed roll 12 continue to rotate for a prescribed time, the compound goes to be transformed into the sheet 37 on the high rotational roll 11 as shown in Figure 5B. Residual resin 37a is formed in the valley-like portion where the high rotational roll 11 meets with the low rotational roll 12. This residue, which is no other than the so-called "bank," is detected by the sensor 76, where the resin is cut. When the pulse motors 16a and 16b are made to rotate at Steps 111 and 113, the end plates 15a and 15b come nearer to or go away from each other, thereby varying the substantial effective width of the kneading rolls 11 and 12. This operation, if repeated, will make the amount of the resin bank 37a appropriate, and completely uniform the kneading of the resin, leading thus to the homogeneous resin sheet for inspection to be manufactured.

When at Step 114 the gap between the rolls 11 and 12 as well as the width between the end plates 15a and 15b are repeatedly widened and narrowed prescribed times, the resin cut shall be terminated. Then at Step 115 the gap between the kneading rolls 11 and 12 shall be matched with a prescribed amount, that is set to the thickness of the resin sheet for inspection to be manufactured by means of the motors 13a and 13b, and at Step 116 the distance between the end plates 15a and 15b shall be matched with prescribed width, that is set to the width of the resin sheet for inspection to be manufactured using the pulse motors 16a and 16b. Under these conditions the kneading rolls 11 and 12 shall further continue to rotate for some time, and then the thickness of the sheet 37 formed on the kneading roll 12 shall be measured. At Step 117 the air cylinder 55 shown in Figure 1 shall be activated to make both the surface displacement meters 53 and 54 advance, when the roller at the top of the surface displacement meter 53 will come in contact with the surface of the sheet 37 to rotate, and the roller at the top of the surface displacement meter 54 will come into direct contact with the kneading roll 11 to rotate. One can know the thickness of the sheet 37 by calculating the difference between the output values of the surface displacement meters 53 and 54. If the results of this measurement permit to make sure that the thickness of the sheet 37 is desired one (Step 118), then the rolls 11 and 12 shall stop rotating at Step 119. Then at Step 120 the motors 13a and 13b shall rotate for parallel displacement of the kneading roll 12. Then the gap between the kneading rolls 11 and 12 shall be widened into the amount almost same with the thickness of the sheet frame 40, and the distance between the end plates 15a and 15b shall be broadened enough at Step 121 by means of the pulse motors 16a and 16b in such a way that the sheet frame 40 can pass freely therethrough. Here the kneading roll 11 shall rotate by inching (Step 122) to displace downward the bank 37a at the valley portion between the kneading rolls 11 and 12. Under the conditions as shown in Figure 5C the sheet frame 40 will be supplied.

As is shown in Figure 4 a number of sheet frames 40, which are housed in the casing 43, are held against the partition plate 41 by the air cylinder 45. In this state the uppermost sheet frame 40 shall be aspirated and held by the upper suction cap 46, then elevated by activating the air cylinder 47. If the air cylinder 52 is activated and the second and subsequent sheet frames 40 are pushed and the air cylinder 45 is made to retreat by the stopper 51, then the second and subsequent sheet frames 40 will go deep into the casing 43. If the stopper 51 is made to retreat by means of the air cylinder 52 and that the lower suction cap 48 is made to aspirate, then the aspiration of the upper suction cap is released, the uppermost sheet frame 40 will be held by the lower suction cap 48. If under this condition the motor 50 is made to rotate, the screw rod 49 will lower down the lower suction cap 48 to bring one of the sheet frames 40 down to the position indicated by the chained lines.

If then the aspiration of the lower suction cap 48 is released, the sheet frame 40 will fall as shown in Figure 5C. Namely this very timing corresponds to Step 123 in the flow chart. The shock given by the falling sheet frame 40 will insert the sheet 37 between the angle of the sheet frame 40 and the kneading roll 11 to cut the sheet 37. Because the sheet 37 is pushed against the sheet frame 40 as shown in Figure 5D if the kneading rolls 11 and 12 are let to rotate at almost same speed (R1 = R2, step 124), the sheet 37 will adhere to the sheet frame 40 from the cut end. Since the kneading rolls 11 and 12 are rotating at almost same speed, the sheet 37 adhered to the sheet free 40 is evacuated amply and smoothly downward as the rotation goes on to fall all along the guide plate 57.

When the sheets 37 and 40 for inspection thus manufactured and fallen are detected, at Step 125, by the sheet No. 1 sensor 72, the belt conveyor 58 shall be driven at Step 126. As is shown in Figure 2 the sheets will be carried over from the guide plate 57 onto the belt conveyor 58 to be transferred onto the set-up table 59 of the sheet for inspection.

When at Step 127 the ends of the sheets 37 and 40 for inspection are detected by the sheet No. 2 sensor 73, the belt conveyor 66 shall be driven (Step 128). At Step 129 the light source 64 shall be illuminated, and at Step 130 the sheets for inspection 37 and 40 shall travel on the set-up table 59 by the belt conveyor 66, activating the video camera 65. Thus the video camera 65 will photograph the fisheyes in the sheet 37 by the light coming through the sheet 37. The number of the fisheyes imaged one after another may be counted with operational circuit and output at the printer to record in memory or else output by a monitor. As soon as the rear ends of the sheets for inspection 37 and 40 are detected by the sheet No. 2 sensor 73 at Step 131, the measurement shall terminate.

If, on the other hand, the falling sheets for inspection 37 and 40 are detected by the sheet No. 1 sensor 72 at Step 125, the air cylinder 21 shall be activated at Step 132 to advance the cleaning roll 22 to clean up the surface of the rolls 11 and 12. At the same time the surface of the endless belt 17 shall be cleaned by the scraper 20 for belt.

Simple introducing thus the compound of the sample resin to be inspected will allow for automatic quantitative inspection of fisheyes caused by non-gelated particles and other foreign matters included in the sample resins.

A Variety of Embodiments

The present invention is not limited to the foregoing embodiments but applicable to others. The objects of the invention will be apparent from the following explanations taken in connection with further modified embodiments about to be described, and various advantages not referred to herein will occur to one skilled in the art upon employment of the invention in practice.

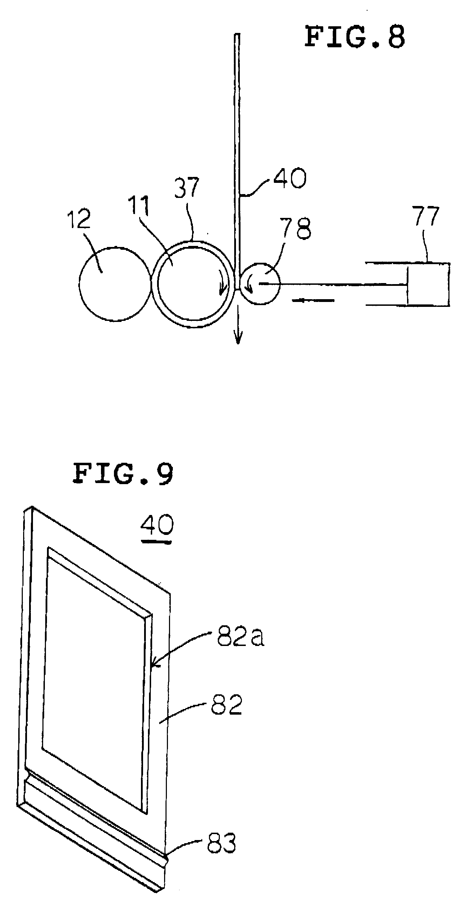

Figure 8 represents an exemplary configuration where the sheet frame 40 is pressed against the sheet 37 on the kneading roll 11 by means of push-down pressure against the roll 11 out of the kneading rolls 11 and 12. The pushing-down means consists, for instance, of an air cylinder 77 and a pinch roller 78. The sheet, when pushed by the pinch roller 78, adheres to the sheet frame 40 to be evacuated downward.

Figure 9 depicts another embodiment of the sheet frame 40 which has, at its central portion, a space for light transmission, and a sheet-cutting slit 83 in the vicinity of the edge of one side of the frame perimeter 82. The sheet frame 40 may have slanted or curved surface at the edge 82a between the light transmission apace and the perimeter 82, regardless of whether the sheet cutting slit 83 exists. The perimeter 82 surface to which the sheet 37 adheres may be applied an adhesive agent.

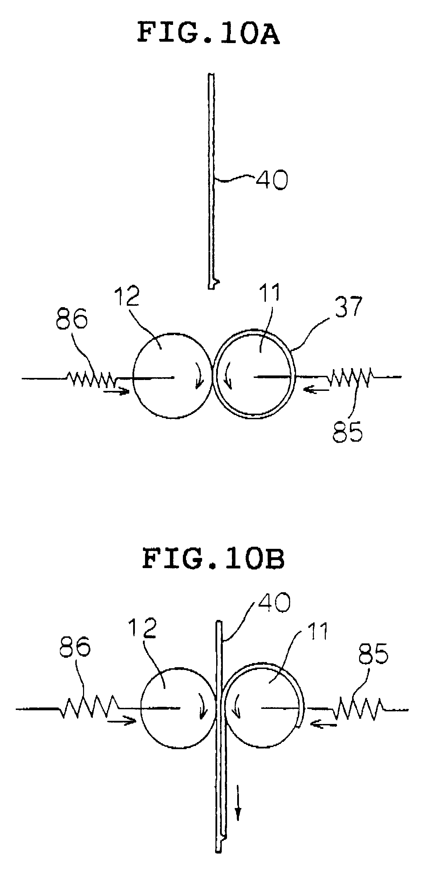

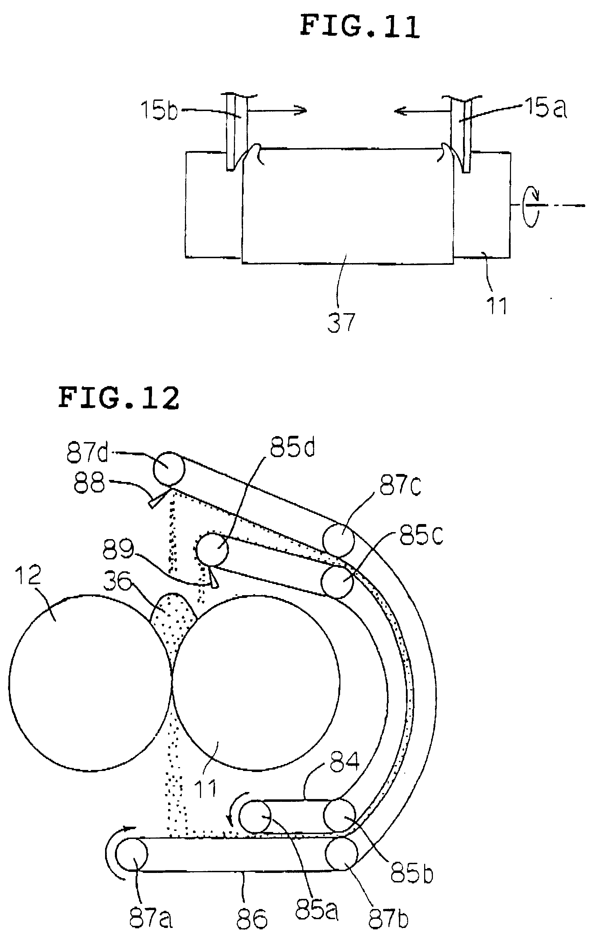

Figures 10A and 10B illustrate embodiments of the invention where the kneading rolls 11 and 12 are pushing against each other by an energizing means. The energizing means used may, for example, be pressure bar springs 85 and 86. If the sheet frame 40 provided, for instance, with such sheet-cutting slit 83 as shown in Fig. 9 is fed between the kneading rolls 11 and 12, thus pressed against each other by the pressure bar springs 85 and 86, then the sheet 37 on the roll 11 will be cut out by the sheet-cutting slit 83 and adhered to the sheet frame 40 to be evacuated downward. This mechanism is feasible also with a sheet frame 40 having no sheet-cutting slit 83, where the sheet 37 on the roll 11 will be cut out at the edge of the lower end of the sheet frame instead. Figure 11 shows an embodiment of the invention where the inner faces of the end plates 15a and 15b are internally curved toward the rotational direction of the kneading roll 11. This configuration makes the kneading of the sheet 37 more through. The inner faces of the end plates 15a and 15b may be flat and narrowly slanted.

Figure 12 represents an embodiment of a device where the resin compound 36 fallen from the fine gap between the kneading rolls 11 and 12 is returned to the valley-formed portion between the same rolls 11 and 12. From below to above the kneading roll 11, the endless belt 84 is wrapped up with the pulleys 85a, 85b, 85c and 85d, and outside of these another endless belt 86 is wrapped up with the pulleys 87a, 87b, 87c and 87d so that they should surround the former belt 84 and pulleys 85a to 85d and rotate in the reverse direction (direction of the arrow) of the pulleys 85a to 85d. The scrapers 89 and 88 come in contact with the lower faces of the upper ends of the endless belt 84 and 86 respectively. The resin compound 36 introduced into the valley portion between the kneading rolls 11 and 12 adheres to the kneading roll 11, a part of which however falls from between the fine gap onto the endless belt 86. This resin compound 36 is held between the two endless belts 84 and 86 to be transported upward, scraped down by the scrapers 89 and 88, then falls again into the valley between the kneading rolls 11 and 12.

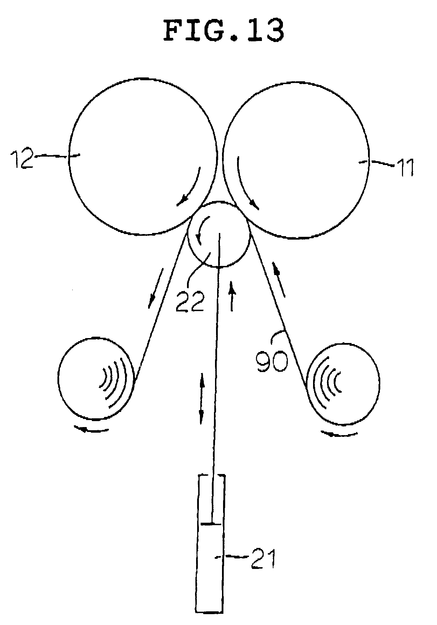

Figure 13 illustrates an exemplary embodiment of the cleaning roll 22 for cleaning the kneading rolls 11 and 12 (refer to Fig. 1) where the slidably contacting face of the cleaning roller 22 coupled with the air cylinder 21 is covered with the cleaning cloth 90. The cleaning cloth 90 is a long strip which is unwound from roll-formed supply source, covers the cleaning roll 22, and then wound up again in roll form, thereby allowing the kneading rolls 11 and 12 always to be in sliding contact with new, clean cloth face. The cleaning cloth 90 may be folded in zigzag form on the supply side.

Industrial Applicability

As has thus far been described the automatic in-resin fisheye inspection apparatus according to this invention allows for any worker, even without any skill whatsoever, to inspect automatically the fisheyes of synthetic resins without resorting to any manpower assistance and any qualification.

**Claims**

1.   An automatic inspection apparatus for fisheyes in a rein comprising
     a pair of rolls for kneading a resin, positioned to shape a fine gap therebetween and a valley where gathering a powder resin supplied from above;
     a driving source for displacing a distance of the gap, linked to an axial support member of at least one of the rolls;
     a pair of end plates for delimiting a substantially effective length of the rolls, formed almost all going along with the valley, nearing but not contacting thereto;
     driving sources for moving the end plates to change the effective length, linked thereto;
     a device for supplying a sheet frame one by one toward the valley;
     a device for carrying an inspecting sheet, which is formed by adhering a resin sheet formed from the powder resin with the operation of the rolls onto the sheet frame at the gap thereof, from a supplying position of the formed inspecting sheet to an inspecting position, and
     a photographing equipment for photographing fisheyes in the inspecting sheet, including a set-up table thereof, a light source and a video camera.

2.   The automatic inspection apparatus according to Claim 1 wherein the photographing equipment is characterized by the set-up table or the unit of the light source and the video camera being linked to a travel driving source to move relatively between the inspecting sheet on the set-up table and the unit thereof.

3.   The automatic inspection apparatus according to Claim 1, further comprising means for pushing pressure a sheet frame to one of the rolls to stick the resin sheet thereunto.

4.   The automatic inspection apparatus according to Claim 3 wherein the means for pushing pressure is a pinch roller.

5.   A manufacturing apparatus of inspecting sheets for inspection of fisheyes in a resin comprising
     a pair of rolls for kneading a resin, positioned to shape a fine gap therebetween and a valley where gathering a powder resin supplied from above;
     a driving source for displacing a distance of the gap, linked to an axial support member of at least one of the rolls;
     a pair of end plates for delimiting a substantially effective length of the rolls formed almost all going along with the valley, nearing but not contacting thereto;
     driving sources for moving the end plates to change the effective length, linked thereto, and
     a device for supplying a sheet frame one by one toward the valley.

6.   The sheet manufacturing apparatus according to Claim 5 further comprising an endless belt having a width almost same with the substantial effective length of the rolls and going its rounds in contact, fro: below the fine gap to above, with one of the rolls, linking to a driving source which displaces the belt at a position in contact with the roll and at another away from the same roll.

7.   The sheet manufacturing apparatus according to Claim 5 further comprising gages for measuring a relative difference between a inner surface of the effective length and a outer surface of thereof one of the rolls.

8.   A sheet frame for adhering to a resin sheet to provide an inspecting sheet characterized in that a central portion have a light transmission apace and that the surround thereof is a plate having a protrusive edge on a face of the surround for cutting off the resin sheet.

9.   The sheet frame according to Claim 8, characterized in that an adhesive agent is applied on one side of the plate.

10.  A device for supplying a sheet frame one by one comprising a pushing pressure means for pressing a number of sheet frames net upright against a partition plate in a casing, a holding means for holding one of the frames on the side of the partition plate, and a means for driving vertically the holding means.

11.  An apparatus for kneading a resin comprising a pair of rolls positioned to shape a fine gap therebetween and a valley where gathering a powder resin supplied from above;

a driving source for displacing a distance of the gap, linked to an axial support member of at least one of the rolls;

a pair of end plates for delimiting a substantially effective length of the rolls, formed almost all going along with the valley, nearing but not contacting thereto;

driving sources for moving the end plates to change the effective length, linked thereto;

a sensor for detecting a resin bank staying along in the valley.

12. The apparatus for kneading a resin according to Claim 11 characterized in that inner faces of said end plates are narrowly slanted toward the rotational direction of the kneading rolls.

13. The apparatus for kneading a resin according to Claim 11 characterized in that inner faces of said end plates are curved inward and toward the rotational direction of the kneading rolls.

14. The apparatus for kneading a resin according to Claim 11 further comprising means for returning a resin, fell from the fine gap between the pair rolls, from below toward up above the rolls.

15. The apparatus for kneading a resin according to Claim 14 characterized in that the means for returning a resin is an endless belt having a width almost some with the substantial effective width of the pair rolls and going its rounds in contact, from below the fine gap, with one of the rolls, being linked with a drive source for displacing the belt from a position in contact with the roll to another position away.

16. The apparatus for kneading a resin according to Claim 11 further comprising a cleaning roll linked to the advance and retreat drive source for contacting with and departing from the lover portions of the pair rolls.

17. An automatically manufacturing method for providing inspecting sheets for inspection of fisheyes in a resin comprising

a step of introducing a powder resin into a valley which is shaped a pair of heated kneading rolls, positioning opposite to each other with a fine gap therebetween and rotating to engulfing direction, rotating speed of one of the pair is high and that of the other is relatively low;

a step of melting and sticking the powder resin onto the high speed roll while receiving and transferring the powder resin fell down through the fine gap to the valley;

a step of varying the clearance of the gap and a substantial effective width in the axial direction of the rolls and adjusting to prescribed clearance and width thereof, and a step of contacting a resin sheet formed, as prescribed thickness and width from the powder resin on the high speed roll, to a sheet frame to adhere thereto.

# FIG.1

# FIG.2

# FIG.3

# FIG.4

# FIG.5A

FIG.5B

FIG.5C

FIG.5D

# FIG.6

| Power supply for light source 64 | C O N T R O L   C I R C U I T | High frequency power supply for rolls 11 and 12 |
|---|---|---|
| Power supply for video camera 65 | | Driving power supply for motors 13a and 13b |
| Trigger for air cylinder 21 | | Driving power supply for motors 16a and 16b |
| Driving power supply for conveyor 58 | | Driving power supply for rolls 11 and 12 |
| Driving power supply for conveyor 66 | | Trigger for air cylinder 18 |
| Compound sensor 71 | | Trigger for air cylinder 55 |
| Sheet No.1 sensor 72 | | Driving circuit for sheet frame supply source 42 |
| Sheet No.2 sensor 73 | | Bank sensor 76 |

80

# FIG.7A

Start

| Temperature rise of rolls 11 and 12 | —101 |

| Initial setting of the gap between rolls 11 and 12 | —102 |

| Initial setting of the width between end plates 15a and15b | —103 |

| Rotation of rolls 11 and 12 $(R_1 > R_2)$ | —104 |

| Advancement of belt 17 | —105 |

| Introduction of compound | —106 |

Detection by sensor 71 —107

Yes

No

| Belt 17 retreats | —108 |

※

# FIG.7B

```
                    ※
                    │
                    ▼
        ┌─────────────────────┐
        │     Waiting for      │──── 109
        │   prescribed time    │
        └─────────────────────┘
                    │
        ┌───────────┘
        │           ▼
        │  ┌─────────────────────┐
        │  │ Width between end   │──── 111
        │  │ plates 15a and 15b  │
        │  │      narrowed       │
        │  └─────────────────────┘
        │           │
        │           ▼
        │  ┌─────────────────────┐
        │  │ Width between end   │──── 113
        │  │ plates 15a and 15b  │
        │  │       widened       │
        │  └─────────────────────┘
        │           │
        │           ▼
        │      ╱─────────────╲
        │     ╱ Prescribed time╲──── 114
   No   └────┤ Prescribed freq. │
             ╲  Away/nearer    ╱
              ╲      ?        ╱
               ╲─────────────╱
                    │ Yes
                    ▼
        ┌─────────────────────┐
        │ Gap between rolls 11 │──── 115
        │   and 12 to the      │
        │  thickness of sheet  │
        └─────────────────────┘
                    │
                    ▼
        ┌─────────────────────┐
        │ Width between end    │──── 116
        │ plates 15a and 15b   │
        │ to the width of sheet│
        └─────────────────────┘
                    │
                    ▼
                  ※  ※
```

# FIG.7C

※ ※

```
┌─────────────────────────────────┐
│   Measurement of thickness      │──── 117
└─────────────────────────────────┘
                │
                ▼
         ╱────────────╲
No  ╱   Thickness of sheet   ╲──── 118
◄──┤                          ├
    ╲        37 ?        ╱
         ╲────────────╱
                │ Yes
                ▼
┌─────────────────────────────────┐
│        Rolls 11 and 12          │──── 119
│        stop rotating            │
└─────────────────────────────────┘
                │
                ▼
┌─────────────────────────────────┐
│   Gap between rolls 11 and      │──── 120
│        12    widened            │
└─────────────────────────────────┘
                │
                ▼
┌─────────────────────────────────┐
│   Width between end plates      │──── 121
│  15a and 15b to be broadened    │
└─────────────────────────────────┘
                │
                ▼
┌─────────────────────────────────┐
│  Inching rotation of roll 11    │──── 122
└─────────────────────────────────┘
                │
123                             │
┌──────────────┐                │
│   Supply of  │────────────────┤
│ sheet frame  │                │
└──────────────┘                │
                │
                ▼
┌─────────────────────────────────┐
│       Rotation of rolls 11      │──── 124
│       and 12 (R₁ ≒ R₂)          │
└─────────────────────────────────┘
                │
                ▼
         ╱────────────╲
No  ╱  Is sheet No. 1 sensor 72  ╲──── 125
◄──┤                              ├
    ╲       detecting ?       ╱
         ╲────────────╱
                │ Yes
```

Rotation of rolls 11 and 12 $(R_1 ≒ R_2)$ ──── 124

※ ※ ※

# FIG.7D

※ ※ ※

132

| Cleaning roll 22 advances |

| Conveyor 58 driven | —126

127 Is sheet No. 2 sensor 73 detecting ?

No

Yes

| Conveyor 66 driven | —128

| Light source 64 be illuminated | —129

| Video camera 65 | —130

131 Is sheet No. 2 sensor 73 detecting ?

No

Yes

Stop

# FIG.8

12  11  37  40  78  77

# FIG.9

40

82a

82

83

20

# FIG.10A

# FIG.10B

# FIG.11

# FIG.12

## FIG.13

INTERNATIONAL SEARCH REPORT

International application No.

PCT/JP94/00309

**A.    CLASSIFICATION OF SUBJECT MATTER**

Int. Cl$^5$  G01N21/89

According to International Patent Classification (IPC) or to both national classification and IPC

**B.    FIELDS SEARCHED**

Minimum documentation searched (classification system followed by classification symbols)

Int. Cl$^5$  G01N21/84-21/90, B29C43/24

Documentation searched other than minimum documentation to the extent that such documents are included in the fields searched

Jitsuyo Shinan Koho          1926 - 1993
Kokai Jitsuyo Shinan Koho    1971 - 1993

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| A | JP, A, 58-108440 (Shin-Etsu Chemical Co., Ltd.), June 28, 1983 (28. 06. 83), (Family: none) | 1-4 |
| A | JP, A, 58-173456 (Showa Electric Wire & Cable Co., Ltd.), October 12, 1983 (12. 10. 83) & DE, A1, 3215067 & US, A, 4692799 | 1-4 |
| A | JP, A, 52-4557 (Matsushita Electric Works, Ltd.), January 13, 1977 (13. 01. 77), (Family: none) | 5-17 |
| A | JP, U, 62-92460  (Mitsubishi Rayon Co., Ltd.), June 12, 1987 (12. 06. 87), (Family: none) | 1-4 |

☐   Further documents are listed in the continuation of Box C.          ☐   See patent family annex.

| | |
|---|---|
| *     Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier document but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | |
| "O"   document referring to an oral disclosure, use, exhibition or other means | "Y"   document of particular relevance; the claimed invention cannot be considered to involve an inventive step when the document is combined with one or more other such documents, such combination being obvious to a person skilled in the art |
| "P"    document published prior to the international filing date but later than the priority date claimed | "&"   document member of the same patent family |

| Date of the actual completion of the international search | Date of mailing of the international search report |
|---|---|
| April 25, 1994 (25. 04. 94) | May 17, 1994 (17. 05. 94) |

| Name and mailing address of the ISA/ | Authorized officer |
|---|---|
| Japanese Patent Office | |
| Facsimile No. | Telephone No. |

Form PCT/ISA/210 (second sheet) (July 1992)